# FASCICULE DE BREVET EUROPEEN

(11) **EP 1 424 993 B1**
(45) Date de publication et mention de la délivrance du brevet: **23.11.2005**
(21) Numéro de dépôt: 02777423.1
(22) Date de dépôt: 11.09.2002
(51) Int. Cl.: A61K 7/48, A61K 35/84

(54) **PROCEDE D'EXTRACTION D'UN PRINCIPE ACTIF RAFFERMISSANT**
VERFAHREN ZUR EXTRAKTION EINES HAUTSTRAFFENDEN WIRKSTOFFS
METHOD FOR EXTRACTING A FIRMING ACTIVE PRINCIPLE, RESULTING ACTIVE PRINCIPLE AND RELATED COMPOSITIONS

(30) Priorité: 12.09.2001 FR 0111780
(43) Date de publication de la demande: 09.06.2004
(73) Titulaire: Société Industrielle Limousine d'Application Biologique (SILAB), 19130 Objat (FR)
(72) Inventeur: PAUFIQUE, Jean-Jacques, F-19130 Objat (FR)
(74) Mandataire: Fantin, Laurent
(86) Numéro de dépôt international: PCT/FR2002/003097
(87) Numéro de publication internationale: WO 2003/022201

(56) Documents cités:
- EP-A- 0 292 601
- EP-A- 0 437 346
- EP-A- 0 600 684
- EP-A- 1 190 627
- FR-A- 2 776 184
- FR-A- 2 797 186
- US-A- 4 032 663

## Description

La présente invention concerne un procédé d'extraction d'un principe actif raffermissant à partir de *Lentinus edodes,* champignon dénommé aussi communément Shiitake, pour le corps, le buste et le visage, le principe actif obtenu et des compositions associées.

Quelles que soient les raisons, on note un vieillissement cutané qui atteint toutes les structures de la peau, notamment la jonction dermo-épidermique.

Cette jonction s'aplatit et de plus, le réseau fibrillaire cutané se dégrade. Ce réseau fibrillaire cutané est aussi connu sous la dénomination matrice extra-cellulaire ou MEC.

Ce réseau fibrillaire, produit par les fibroblastes et les kératinocytes, est composé de macromolécules, principalement des fibres de collagène et l'élastine. Ce réseau fibrillaire comprend ainsi des fibres insolubles, rigides et stables, qui à partir de leur organisation spatiale confèrent aux tissus conjonctifs leur solidité et leur résistance mécanique.

En plus de ces fonctions mécaniques, ce réseau présente des fonctions biologiques d'échange et de communication intercellulaire permettant un bon ancrage épiderme/derme et une intégrité du tissu cutané.

Dans les conditions normales, à savoir sur une peau jeune, ce réseau est soumis à un équilibre métabolique qui est géré par des enzymes spécifiques, les métalloprotéinases ou MMPs, qui sont capables de dégrader différents composants de la matrice extracellulaire. L'activité de ces enzymes est contrôlée en partie par des inhibiteurs tissulaires spécifiques appelés TIMPs (Tissue Inhibitor of Matrixproteinase).

Ainsi, les fibres de collagène vieillies et déstructurées sont remplacées au fur et à mesure, tant que l'équilibre perdure.

Les conditions de stress environnemental auxquelles est soumise la peau, comme l'exposition aux UVA et UVB, ainsi que le vieillissement naturel, détruisent cet équilibre provoquant une diminution de l'activité métabolique cellulaire, donc une diminution du renouvellement des fibres de collagène.

L'activité des métalloprotéinases s'emballe et les inhibiteurs tissulaires ne contrôlent plus cette activité.

Les fibres de collagène sont dégradées en grand nombre sans être remplacées et ceci de façon anarchique, ce qui conduit à une déstructuration de la matrice extra-cellulaire.

La peau perd de sa souplesse, de son élasticité et elle s'affaisse.

Pour éviter une telle déstructuration et améliorer les propriétés biomécaniques de la peau, la solution proposée par la présente invention est de contrôler l'activité des MMPs.

Or dans le groupe des métalloprotéinases de la matrice, on trouve un groupe d'une vingtaine d'enzymes Zinc dépendantes, plus particulièrement deux groupes, celui des collagénases et des gélatinases.

On s'intéresse dans la présente invention essentiellement au contrôle de l'activité de la collagénase-1 ou MMP-1 qui dégrade plus spécifiquement les collagènes de type I et III. Son effet est particulièrement sensible suite au photo-vieillissement.

Dans le groupe des gélatinases dont on sait qu'elles sont impliquées dans le remodelage des collagènes de la matrice extra-cellulaire, on s'intéresse plus particulièrement à la Gélatinase A ou MMP-2. En effet, cette enzyme dégrade essentiellement les fibres de collagène I et hydrolyse aussi les collagènes III, IV et VII spécifiques de la jonction dermo-épidermique, l'élastine et la fibronectine.

L'inhibiteur TIMP-1 agit sur les MMP-1 notamment et l'inhibiteur TIMP-2 agit notamment sur la MMP-2.

Le document brevet EP-A-1 190 627 décrit un procédé d'extraction d'un principe actif, caractérisé en ce que en ce qu'il comprend les étapes suivantes:
- solubilisation de champignons broyés,
- hydrolyse enzymatique en présence d'une protéinase
- séparation des phases soluble et insoluble.

Le pH préféré est de 5.0 à 7.0.

A cet effet, le procédé selon la présente invention concerne un procédé d'extraction d'un principe actif à partir de *Lentinus edodes*, riche en oligosaccharides et en acides uroniques afin d'inhiber l'activité de certaines métalloprotéinases.

L'invention couvre aussi le principe actif obtenu et des compositions adaptées.

Le procédé de la présente invention est maintenant décrit de façon détaillé suivant un mode de réalisation, accompagné de la caractérisation du principe actif obtenu et des résultats sur cellules et sur volontaires suite à des applications de compositions contenant le principe actif.

### 1/PROCEDE D'EXTRACTION D'UN PRINCIPE ACTIF A PARTIR DE LENTINUS EDODES

Le procédé d'extraction du principe actif selon la présente invention consiste en la succession des étapes suivantes :
- solubilisation de *Lentinus edodes* à au moins 20 g/l en milieu légèrement alcalin,
- hydrolyse enzymatique en présence d'une protéase à pH basique,
   - séparation des phases soluble et insoluble par tout moyen ou toute combinaison de moyens, par exemple : décantation, filtration ou centrifugation,
   - concentration de la phase active,
   - purification de la fraction sucre, notamment oligosaccharides et acides uroniques, et
- filtration stérilisante pour atteindre une flore mésophile totale inférieure à 100 germes /g, et une absence de levures et de moisissures.

### 2/ CARACTERISATION DU PRINCIPE ACTIF OBTENU

### Taux de matières sèches :

Les matières sèches sont obtenues par passage du principe actif extrait dans une étuve à 105°C pendant 16 heures. La quantité de matières sèches obtenue est comprise entre 20 et 200 g/l, plus particulièrement comprise entre 50 et 100 g/l.

### Mesure du pH :

Le pH est mesuré par la méthode potentiométrique à température ambiante. Le pH est compris entre 4 et 10, plus particulièrement entre 5 et 10.

### Détermination de la teneur en protéines :

La détermination est réalisée par la méthode de Lowry.

La teneur en protéines est comprise entre 10 et 100 g/l et plus particulièrement entre 10 et 50 g/l.

### Détermination de la teneur en sucres totaux :

Cette détermination est réalisée par la méthode de Dubois (DUBOIS, M & al, [1956], Analytical chemistry, 28, N°3 p 350-356).

Les sucres réducteurs donnent un composé coloré en présence de phénol et d'acide sulfurique. On lit la densité optique de la composition colorée et on lit le taux de sucres sur une courbe étalon réalisée dans les mêmes conditions avec une composition étalon à 1/3 de mannose, 1/3 de glucose et 1/3 de galactose sur une plage de 25 à 100 µg/ml.

Le taux de sucres totaux est compris entre 5 et 50 et plus particulièrement entre 5 et 20 g/l.

### Dosage des acides galacturoniques:

Les acides uroniques sont les constituants d'unités répétitives spécifiques à tous les polysaccharides acides.

Ces acides galacturoniques sont notamment les marqueurs des polysaccharides du type pectines.

On peut les détecter par une analyse comparée de coloration, par rapport à une gamme étalon d'acides galacturoniques.

On obtient pour le principe actif selon la présente invention un taux de 0,5 g/l

### Chromatoaraphie des degrés de polymérisation des oligosaccharides :

Cette courbe montre que la majorité des oligosaccharides présentent un degré de polymérisation inférieur ou égal à 5 unités monosaccharides.

### 3/ EFFET DU PRINCIPE ACTIF SUR L'ACTIVITE DE MMP-1

On incube des fibroblastes humains avec le principe actif.

Certains échantillons sont soumis à un traitement à une irradiation par des UVA à une puissance de 20 joules/cm² pour donner un ordre de grandeur et d'autres à aucune irradiation.

Le surnageant cellulaire permet de réaliser le dosage du taux de MMP-1 par méthode ELISA.

Le taux est exprimé en ng/ml.

Le tableau de la figure 1 montre les résultats obtenus.

La variation Δ% est obtenue en comparant les taux obtenus pour l'échantillon traité par le principe actif et celui du témoin.

Le principe actif selon la présente invention provoque une inhibition de la synthèse des MMP-1, ceci de façon dose-dépendante.

Plus particulièrement, on note que cette inhibition est vérifiée dans des conditions de stress particulièrement agressives engendrées par l'irradiation.

### 4/ EFFET DU PRINCIPE ACTIF SUR L'ACTIVITE DE MMP-2

Le protocole opératoire vise à effectuer une étude immunohistochimique qui permet d'obtenir des résultats quantitatifs.

A cet effet, on réalise un immunomarquage sur les surnageants de culture de fibroblastes humains mis en présence de principe actif.

On choisit :
- un anticorps primaire, un anticorps monoclonal de souris anti-humain MMP-2,
- comme anticorps secondaire, un anticorps anti-IgG de souris,
- un système de révélation.

La visualisation est réalisée au moyen d'un microscope relié à un analyseur d'images.

Les résultats sont représentés.

On constate une diminution de la synthèse des MMP-2, ceci de façon dose-dépendante.

### 5/ EFFET DU PRINCIPE ACTIF SUR L'ACTIVITE TIMP-1

Cette étude vise à évaluer l'effet du principe actif sur l'activité des TIMP-1

On réalise le dosage du taux de TIMP-1 par méthode ELISA dans des surnageants de cultures de fibroblastes humains témoin d'une part et mis en présence de principe actif d'autre part.

Ces surnageants avec et sans principe actif sont testés directement tandis que d'autres subissent une irradiation par des UVB avec une puissance de 0,25 J/cm² .

Le taux d'activité est exprimé en ng/ml et les résultats sont regroupés.

On constate que le principe actif selon la présente invention provoque une augmentation de la synthèse des TIMP-1, ceci plus particulièrement dans des conditions de stress engendrées par irradiation.

### 6/ CONTRIBUTION DU PRINCIPE ACTIF SUR LE RAFFERMISSEMENT DE LA PEAU

L'étude porte sur les propriétés biomécaniques de la peau, à savoir l'élasticité, la tonicité et la fatigabilité.

Le principe actif est introduit dans une émulsion à 5% et les effets sont quantifiés sur deux groupes, l'un A de 10 volontaires d'âges compris entre 24 et 40 ans, l'autre B de 15 volontaires d'âges compris entre 45 et 70 ans, avant et après 28 jours d'applications biquotidiennes de cette émulsion et du placebo pendant 28 jours sur l'avant-bras. Dans les mêmes conditions, le test est réalisé sur le buste d'un groupe C de 10 volontaires d'âge compris entre 24 et 57 ans.

Les mesures sont effectuées au moyen d'un appareil de mesure du commerce, connu sous la dénomination Cutomètre SEM 575.

Un tel appareil dispose d'une sonde avec un orifice soumis à une dépression constante pendant une durée constante.

Les courbes obtenues permettent de déterminer les paramètres suivants
- L'élasticité de la peau.
- La tonicité de la peau.
- La fatigabilité.

La synthèse des résultats est indiquée pour les avant-bras et bustes des volontaires des groupes A, B et C.

On constate une augmentation de l'élasticité, d'autant plus prononcée pour les peaux du groupe B qui correspondent à des peaux matures, de la tonicité et une diminution de la fatigabilité au niveau des avant-bras. Ceci est confirmé par les tests sur le buste.

## Revendications

1. Procédé d'extraction d'un principe actif à visée cosmétique, **caractérisé en ce que en ce qu'**il comprend les étapes suivantes :
- solubilisation en milieu légèrement alcalin de champignons *Lentinus edodes* entiers, broyés, avec une proportion d'au moins 20 g/l
- hydrolyse enzymatique en présence d'une protéase à pH basique,
- séparation des phases soluble et insoluble par tout moyen ou toute combinaison de moyens, par exemple : décantation, filtration ou centrifugation,
- filtration stérilisante pour atteindre une flore mésophile totale inférieure à 100 germes /g, et une absence de levures et de moisissures.

2. Procédé d'extraction selon la revendication 1 d'un principe actif à visée cosmétique, **caractérisé en ce qu'**il comprend une étape supplémentaire de purification de la fraction sucre, notamment oligosaccharides et acides uroniques.

3. Principe actif obtenu suivant le procédé de la revendication 1, permettant d'inhiber l'activité des métalloprotéinases, notamment MMP-1 et MMP-2 à la jonction dermo-épidermique, **caractérisé en ce qu'**il présente les paramètres suivants :
- taux de matières sèches obtenu à 105°C par passage à l'étuve pendant 16 heures compris entre 20 et 200g/l,
- pH compris entre 5,0 et 10,0,
- teneur en protéines déterminée par la méthode de Lowry comprise entre 10 et 100 g/l,
- teneur en sucres totaux déterminée par la méthode de Dubois comprise entre 5 et 50 g/l.

4. Principe actif selon la revendication 3, **caractérisé en ce qu'**il présente les paramètres suivants :
- taux de matières sèches obtenu à 105°C par passage à l'étuve pendant 16 heures compris entre 50 et 100g/l,
- teneur en protéines déterminée par la méthode de Lowry comprise entre 10 et 50 g/l,
- teneur en sucres totaux déterminée par la méthode de Dubois comprise entre 5 et 20 g/l.

5. Principe actif obtenu suivant le procédé de la revendication 1, permettant d'accroître la synthèse des TIMP-1, **caractérisé en ce qu'**il présente les paramètres suivants :
- taux de matières sèches obtenu à 105°C par passage à l'étuve pendant 16 heures compris entre 20 et 200g/l,
- pH compris entre 5,0 et 10,0,
- teneur en protéines déterminée par la méthode de Lowry comprise entre 10 et 100 g/l,
- teneur en sucres totaux déterminée par la méthode de Dubois comprise entre 5 et 50 g/l.

6. Principe actif selon la revendication 5, **caractérisé en ce qu'**il présente les paramètres suivants :
- taux de matières sèches obtenu à 105°C par passage à l'étuve pendant 16 heures compris entre 50 et 100g/l,
- teneur en protéines déterminée par la méthode de Lowry comprise entre 10 et 50 g/l,
- teneur en sucres totaux déterminée par la méthode de Dubois comprise entre 5 et 20 g/l.

7. Principe actif obtenu suivant le procédé de la revendication 1, permettant d'obtenir une raffermissement de la peau, **caractérisé en ce qu'**il présente les paramètres suivants :
- taux de matières sèches obtenu à 105°C par passage à l'étuve pendant 16 heures compris entre 20 et 200g/l,
- pH compris entre 5,0 et 10,0,
- teneur en protéines déterminée par la méthode de Lowry comprise entre 10 et 100 g/l,
- teneur en sucres totaux déterminée par la méthode de Dubois comprise entre 5 et 50 g/l.

## Claims

1. A method of extracting an active principle for cosmetic purposes, **characterised in that** it comprises the following steps:
- solubilisation, in a slightly alkaline medium, of ground whole *Lentinus* edodes mushrooms, with a proportion of at least 20 g/l,
- enzymatic hydrolysis in the presence of a basic pH protease,
- separation of the soluble and insoluble phases by any means or any combination of means, for example: settling, filtration or centrifugation,
- sterilising filtration in order to achieve a total mesophilic flora of less than 100 germs/g, and an absence of yeasts and moulds.

2. A method of extracting an active principle for cosmetic purposes according to claim 1, **characterised in that** it comprises a supplementary step of purification of the sugar fraction, in particular oligosaccharides and uronic acids.

3. An active principle obtained by the method of claim 1, enabling inhibition of the activity of the metalloproteinases, in particular MMP-1 and MMP-2 at the dermo-epidermic junction, **characterised in that** it has the following parameters:
- proportion of dry matter obtained at 105°C by passing through a stove for 16 hours of between 20 and 200 g/l,
- pH between 5.0 and 10.0,
- protein content determined by the Lowry method of between 10 and 100 g/l,
- total sugar content determined by the Dubois method of between 5 and 50 g/l.

4. An active principle according to claim 3, **characterised in that** it has the following parameters:
- proportion of dry matter obtained at 105°C by passing through a stove for 16 hours of between 50 and 100 g/l,
- protein content determined by the Lowry method of between 10 and 50 g/l,
- total sugar content determined by the Dubois method of between 5 and 20 g/l.

5. An active principle obtained by the method of claim 1, enabling an increase in the synthesis of TIMP-1, **characterised in that** it has the following parameters:
- proportion of dry matter obtained at 105°C by passing through a stove for 16 hours of between 20 and 200 g/l,
- pH between 5.0 and 10.0,
- protein content determined by the Lowry method of between 10 and 100 g/l,
- total sugar content determined by the Dubois method of between 5 and 50 g/l.

6. An active principle according to claim 5, **characterised in that** it has the following parameters:
- proportion of dry matter obtained at 105°C by passing through a stove for 16 hours of between 50 and 100 g/l,
- protein content determined by the Lowry method of between 10 and 50 g/l,
- total sugar content determined by the Dubois method of between 5 and 20 g/l.

7. An active principle obtained by the method of claim 1, enabling a firming of the skin, **characterised in that** it has the following parameters:
- proportion of dry matter obtained at 105°C by passing through a stove for 16 hours of between 20 and 200 g/l,
- pH between 5.0 and 10.0,
- protein content determined by the Lowry method of between 10 and 100 g/l,
- total sugar content determined by the Dubois method of between 5 and 50 g/l.

## Patentansprüche

1. Verfahren zur Extraktion eines Wirkstoffes für kosmetische Zwecke, **dadurch gekennzeichnet, dass** es die folgenden Schritte umfasst:
- Auflösen von ganzen, zerkleinerten Pilzen *Lentinus edodes* in einem leicht alkalischen Medium mit einem Anteil von mindestens 20 g/l,
- enzymatische Hydrolyse in Gegenwart einer Protease mit basischem pH-Wert,
- Trennen der löslichen und unlöslichen Phasen durch irgendein Mittel oder irgendeine Kombination von Mitteln, beispielsweise: Dekantieren, Filtern oder Zentrifugieren,
- sterilisierendes Filtern, um eine mesophile Gesamtflora mit weniger als 100 Keimen/g und ein Fehlen von Hefe- und Schimmelpilzen zu erhalten.

2. Verfahren nach Anspruch 1 zur Extraktion eines Wirkstoffes für kosmetische Zwecke, **dadurch gekennzeichnet, dass** es einen zusätzlichen Schritt der Reinigung der Zuckerfraktion, insbesondere der Oligosaccharide und Uronsäuren, umfasst.

3. Wirkstoff, der nach dem Verfahren aus Anspruch 1 erhalten wird und es ermöglicht, die Aktivität der Metallproteinasen, insbesondere MMP-1 und MMP-2, an der dermo-epidermischen Verbindung zu hemmen, **dadurch gekennzeichnet, dass** er die folgenden Parameter aufweist:
- Trockenmaterialanteil, erhalten bei 105 °C im Trockenofen 16 Stunden lang, zwischen 20 und 200 g/l,
- pH-Wert zwischen 5,0 und 10,0,
- Gehalt an Proteinen, bestimmt durch die Lowry-Methode, zwischen 10 und 100 g/l,
- Gesamtzuckergehalt, bestimmt durch die Dubois-Methode, zwischen 5 und 50 g/l.

4. Wirkstoff nach Anspruch 3, **dadurch gekennzeichnet, dass** er die folgenden Parameter aufweist:
- Trockenmaterialanteil, erhalten bei 105 °C im Trockenofen 16 Stunden lang, zwischen 50 und 100 g/l,
- Gehalt an Proteinen, bestimmt durch die Lowry-Methode, zwischen 10 und 50 g/l,
- Gesamtzuckergehalt, bestimmt durch die Dubois-Methode, zwischen 5 und 20 g/l.

5. Wirkstoff, der nach dem Verfahren aus Anspruch 1 hergestellt wird und es ermöglicht, die Synthese der TIMP-1 zu steigern, **dadurch gekennzeichnet, dass** er die folgenden Parameter aufweist:
- Trockenmaterialanteil, erhalten bei 105 °C im Trockenofen 16 Stunden lang, zwischen 20 und 200 g/l,
- pH-Wert zwischen 5,0 und 10,0,
- Gehalt an Proteinen, bestimmt durch die Lowry-Methode, zwischen 10 und 100 g/l,
- Gesamtzuckergehalt, bestimmt durch die Dubois-Methode, zwischen 5 und 50 g/l.

6. Wirkstoff nach Anspruch 5, **dadurch gekennzeichnet, dass** er die folgenden Parameter aufweist:
- Trockenmaterialanteil, erhalten bei 105 °C im Trockenofen 16 Stunden lang, zwischen 50 und 100 g/l,
- Gehalt an Proteinen, bestimmt durch die Lowry-Methode, zwischen 10 und 50 g/l,
- Gesamtzuckergehalt, bestimmt durch die Dubois-Methode, zwischen 5 und 20 g/l.

7. Wirkstoff, der nach dem Verfahren aus Anspruch 1 hergestellt wird und es ermöglicht, eine Straffung der Haut zu erzielen, **dadurch gekennzeichnet, dass** er die folgenden Parameter aufweist:
- Trockenmaterialanteil, erhalten bei 105 °C im Trockenofen 16 Stunden lang, zwischen 20 und 200 g/l,
- pH-Wert zwischen 5,0 und 10,0,
- Gehalt an Proteinen, bestimmt durch die Lowry-Methode, zwischen 10 und 100 g/l,
- Gesamtzuckergehalt, bestimmt durch die Dubois-Methode, zwischen 5 und 50 g/l.
